(19) Europäisches Patentamt
European Patent Office
Office européen des brevets

(11) Publication number: **0 188 793**
**A1**

# (12) EUROPEAN PATENT APPLICATION

(21) Application number: **85116389.9**

(22) Date of filing: **20.12.85**

(51) Int. Cl.⁴: **A 61 K 7/06**, A 61 K 31/505

(30) Priority: **21.12.84 US 684736**

(43) Date of publication of application: **30.07.86 Bulletin 86/31**

(84) Designated Contracting States: **AT BE CH DE FR GB IT LI LU NL SE**

(71) Applicant: **Richardson-Vicks, Inc., Ten Westport Road, Wilton, CT 06897 (US)**

(72) Inventor: **Gans, Eugene H., 5 Fairview Drive, Westport Connecticut 06880 (US)**
Inventor: **Yeung, David, 31 Windermere Lane, Stamford Connecticut 06902 (US)**

(74) Representative: **Vossius Vossius Tauchner Heunemann Rauh, Siebertstrasse 4 P.O. Box 86 07 67, D-8000 München 86 (DE)**

(54) **Improved topical minoxidil composition.**

(57) Hydroalcoholic solutions of minoxidil for topical use improved by addition of hydroxy (lower alkyl) arcylate polymer or hydroxy (lower alkyl) methacrylate polymer.

EP 0 188 793 A1

Our Ref.: U 198 EP
Case: V-1293 EP
Richardson-Vicks Inc.

HEINEMANN · RAUH
PATENTANWÄLTE
SIEBERTSTR. 4, 8000 MÜNCHEN 80
TEL. (089) 47 4075

0188793

December 29, 1985

## IMPROVED TOPICAL MINOXIDIL COMPOSITION

### Field of the Invention

This invention relates to improved topical compositions containing minoxidil for use in regenerating hair growth.

### Background of the Invention

Minoxidil is the generic name for 2,4-diamino-6-piperidino-pyrimidine-3-oxide, a potent orally administered peripheral vasodilator that is used to control severe hypertension in patients who are resistant to conventional therapy. In recent years, articles in the literature have appeared reporting on observed hair regrowth in hypertensive patients receiving oral minoxidil therapy. For example, see Zappacosta A.R.: "Reversal of baldness in patient receiving minoxidil for hypertension", N. Engl. J. Med. 1980; 303: 1480-81, which reports on the reversal of male-pattern alopecia (i.e., loss of hair on the head; baldness) in a patient receiving oral minoxidil therapy for hypertension. In 1981, Weiss et al in J. Am. Acad. Dermatol. 1981; 5: 224-26, reported preliminary observations in which patients treated with a 1% topical minoxidil solution demonstrated hair regrowth at sites of severe, treatment-resistant alopecia areata; and in 1984, Weiss et al in Arch. Dermatol. 1984; 120: 457-463, reported on further testing of 1% minoxidil topical solutions on patients with alopecia areata with significant hair regrowth results. Such studies have drawn the attention of dermatologists to minoxidil as an active

research candidate and potential therapeutic entity for hair regrowth and the treatment of alopecia areata. The instant invention provides an improved formulation for such research and therapeutic purposes.

## Detailed Description of Invention

The instant invention relates to an improved hydroalcoholic composition of minoxidil for topical hair regrowth applications such as alopecia areata. In prior art studies (Weiss et al, ibid.), aqueous ethanolic solutions were utilized such as, for example, a 1% minoxidil solution in a vehicle containing approximately 70% alcohol, 20% water and 10% propylene glycol. The minoxidil solution was applied by a glass rod applicator at the site of hair loss. The high fluidity of such solutions, and of other similarly fluid scalp or hair-growth treatment products, however, presents the problem of seepage to unwanted areas where hair regrowth may be undesirable. Such problem is substantially eliminated upon use of the subject composition.

In accordance with this invention, there is provided an improved hydroalcoholic composition containing an effective hair regrowth amount of minoxidil. In general, the composition is a solution of from about 0.5 to about 5.0 w/v percent, and preferably from about 1 to about 3 w/v percent, of minoxidil in an aqueous ethanolic solvent comprising from about 20 to about 80 v/v percent of ethanol, preferably from about 40 to about 60 percent of ethanol, and most preferably about 50 percent ethanol, the improvement comprising the inclusion of from about 1 to about 10 w/v percent, and preferably from about 3 to about 5 percent, of hydroxy (lower alkyl) acrylate polymer or hydroxy (lower alkyl)

methacrylate polymer or mixtures thereof. The addition of up to about 10% w/v percent of propylene glycol is optional.

Said homopolymers are linear without substantial cross-linking (i.e., the cross-linking should not be sufficient to render the polymer insoluble in the hydroalcoholic solvent). The term "lower alkyl" denotes 2 or 3 carbons. Typical such polymers include, for example, those obtained by the polymerization of such hydrophilic monomers as 2-hydroxyethyl acrylate, 2-hydroxyethyl methacrylate (preferred), 2-hydroxypropyl acrylate, 3-hydroxypropyl methacrylate and the like.

The hydroxy (lower alkyl) acrylate and methacryllate polymers utilized herein and their methods of preparation are well known and the polymers are readily available commercially. Their usage in cosmetic preparations is also well known, for example, see Canadian Patent No. 945,474 and British Patent No. 1,261,433.

The subject linear homopolymers of hydroxy (lower alkyl) acrylate and hydroxy (lower alkyl) methacrylate are advantageously used as globular particles with average diameter of 0.1 to 2 millimeters. Preferably, the diameter is about 20-100 microns and even less than 10 microns, the latter size being suitably provided by following the methodology, as applied to the aforementioned monomeric polymerization, described in U.S. Patent No. 3,583,957 for the copolymerization of olefins to produce globular particles having a diameter of less than ten microns.

The preferred hydroxyethyl methacrylate polymers are embraced by the formula:

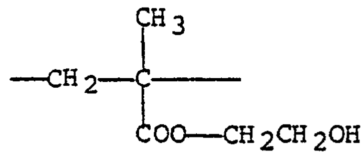

(I)

wherein n is the number of recurring monomeric units ranging from about 70 to about 400. Such hydrophilic homopolymers are soluble in alcohol and water-alcohol mixtures. A particularly preferred commercial polymer product of Formula (I) is available as a white powder from National Patent Development Corp., New Brunswick, N.J. under its trademark "Hydron-N".

Although aqueous ethanolic solutions are preferred, aqueous solutions of isopropanol may also be beneficially employed as solvents herein.

As noted previously, one of the potential problems associated with the application of a scalp or hair growth treatment product is the seepage, or drippage, of the product to unwanted areas, that is, other than the treatment site. This may result in the regrowth or stimulated growth of hair in such other areas as, for example, eyelashes, eyebrows and terminal facial hair, due to the undesirable action of the particular hair regrowth active ingredient at such sites.

It has now been found that the incorporation of hydroxy (lower alkyl) acrylate polymer, hydroxy (lower alkyl) methacrylate polymer or mixtures thereof, preferably hydroxyethylmethacrylate polymer of Formula (I), into topical hydroalcoholic compositions containing an effective hair growing amount of minoxidil effectively retards the seepage of the product beyond the application site and consequently localizes the activity of said minoxidil to the treatment area. In

addition, such compositions are effective in providing skin penetration of minoxidil at the treated site.

The improved composition of this invention, therefore, provides the dermatologist and the ultimate user with a unique composition which is highly effective in confining and localizing the composition to the treatment area without excessive seepage and in delivering the active hair regrowth ingredient, minoxidil, for effective skin penetration. The subject compositions are stable and readily prepared in accordance with general formulating methodology within the skill of the art.

The following examples are presented to illustrate but not to limit the subject invention.

## Example 1

This example demonstrates the effectiveness of the hydroxyethylmethacrylate polymer of Formula (I) in retarding seepage when incorporated into a hydroalcoholic vehicle. An in-vitro assay is utilized which measures the distance traveled by a 10 µl drop of the test formulation down a glass plate inclined at 45°C for 10 seconds. The test formulation is colored with dye to enhance the visual movement of the product. Pure ethanol (Ethanol, U.S.P) is used as the standard against which the test formulation is compared. In addition to this Formula (I) polymer, formulations of other polymers and gums with similar viscosities (measured on a Brookfield LVT Viscometer) are also tested for comparative purposes. The solvent system for each test formulation is 50:50 EtOH:HOH.

The sodium carboxymethylcellulose (Na-CMC) utilized was cellulose gum No. 12M31, commercially available from Hercules Inc., Wilmington, Delaware. Xanthan gum, a high molecular weight natural polysaccharide, was obtained from Kelco, a division of Merck & Co., Inc., Rahway, N.J. The term "Carbomer" is the official designation used in the CFTA Cosmetic Ingredient Dictionary for the acrylic acid polymeric resins commercially available under the trademark "Carbopol" of B.F. Goodrich, Cleveland, Ohio. Carbomer 940 has an approximate molecular weight of 4,000,000. The particular hydroxyethylmethacrylate polymer utilized herein is the previously identified commercial product, "Hydron-N".

The ratio of the distance traveled ("dripping distance") by the particular test formulation to that of the ethanol standard provides the "dripping index" number for the test formulation.

Table I

Relative Dripping Distance of Various Formulations

| Treatment | Viscosity (Centipoise) | Dripping Distance (mm) | Dripping Index |
|---|---|---|---|
| 100% Ethanol | | 60 | 1.00 |
| 50:50 EtOH:HOH | 2.7 | 49 | 0.82 |
| 0.2% Sodium carboxy-methylcellulose* | 8.4 | 47 | 0.78 |
| 0.02% Xanthan gum | 9.0 | 42 | 0.70 |
| 0.2% Carbomer 940 resin* | 8.6 | 36 | 0.60 |
| 3% Hydroxyethylmeth-acrylate polymer | 8.9 | 34 | 0.57 |
| 0.2% Sodium carboxy-methylcellulose* | 14 | 36 | 0.60 |
| 0.05% Xanthan gum | 15 | 34 | 0.57 |
| 0.02% Carbomer 940 resin* | 15 | 30 | 0.50 |
| 5% Hydroxyethylmeth-acrylate polymer | 14 | 27 | 0.45 |

*Viscosity adjusted by varying pH of solution

The indicated data in Table I shows that hydroxyethylmethacrylate polymer, of the agents tested in 50:50 aqueous ethanolic solutions of comparable viscosity, is most effective in controlling dripping (i.e., seepage). The 3% solution (dripping index = 0.57) and the 5% solution (dripping index = 0.45) affords an effective retardation of seepage of about 30% and 45%, respectively, over that obtained by the 50:50 EtOH:HOH control (dripping index = 0.82).

Example 2

This example demonstrates the drug release characteristics of the subject polymers by use of an in vitro artificial membrane system whose permeability appears to correlate with that of human skin, thereby reducing the need for excised human skin as a screening device (Dermatology Times, Vol. 4, No. 7, July 1983). To a hydroalcoholic (50:50 EtOH:HOH) solution containing 0.5% w/v minoxidil is added 1% w/v polymer (Hydron-N). Similar solutions of other thickening agents indicated in Table II are also used for comparative purposes. In this study, a membrane diffusion cell set-up is utilized to measure drug release characteristics of the test formulations. The membrane used is an artificial membrane system composed of an approximately 700 microns (1 micron = 0.001 mm) thick, three-layered membrane wherein the inner layer consists of siliconized rubber, i.e., a dimethyl polysiloxane membrane, and the two outer layers are each a membrane made of cellulose acetate annealed by heating to 60°C. The dimethyl polysiloxane membrane, sandwiched between the two cellulose acetate membranes, is Silastic® Medical Grade dimethyl polysiloxane nonreinforced sheeting (0.127 mm i.e. 0.005 inch thick) supplied by Dow-Corning Corp., Medical Products Division, Midland, MI. The cellulose acetate membranes are supplied by V.O.P. Inc., Fluid Systems Division, San Diego, CA, with the following specification: 0.0086 inch (0.22 mm) wet thickness and with annealing temperature of 60°C. The membrane system, 2 inches (50.8 mm) in diameter, is mounted between two cell compartments of a diffusion cell apparatus (see figures in Dermatology Times, supra). This apparatus

is composed of two compartments: the lower chamber made of thermoplastic poly(methyl methacrylate) polymer (Plexiglas®, Rohm & Haas Co., Philadelphia, Penn.) and the top made of tetrafluoroethylene polymer resin (Teflon®, Du Pont, Wilmington, Del.). The membrane system is mounted with one side exposed to the air and the other side constantly bathed by filling the receptor compartment with phosphate buffer solution of pH 7.2. This diffusion set-up provides an effective diffusional area of 8.03 $cm^2$. Adequate mixing of the receptor solution is accomplished by stirring with a magnetic bar at constant speed (120 rpm). The whole diffusion cell assembly is immersed in a water bath maintained at 30°C by a thermostatic pump. 0.5 Milliliter of the test product is accurately measured and applied to the membrane surface which is exposed to the air. The drug is then diffused through the membrane system into the receptor buffer and the rate of drug diffusion is dependent on the effectiveness of the vehicle to release the drug for penetration. At selected time intervals (hourly) after product application, an aliquot of the receptor buffer solution is withdrawn and analyzed by UV spectrophotometer (Varian model 210 UV-Visible spectrophotometer) for drug content. Table II summarizes the cumulative transfer of minoxidil from each of the indicated systems over a period of five hours. Results are expressed as the rate of minoxidil transferred through the membrane system (rate = micrograms per $cm^2$ per hour) as defined by the amount of minoxidil (micrograms) transferred through the membrane area ($cm^2$) over a defined period of time (hours).

Table II

Release of Minoxidil from Various Systems

| Time (Hours) | CUMULATIVE TRANSFER (micrograms) Hydroxyethyl Methacrylate | Sodium Carboxy methylcellulose | Xanthan Gum | Carbomer 940 Resin |
|---|---|---|---|---|
| 1 | 181 | 127 | 130 | 32 |
| 2 | 327 | 225 | 225 | 87 |
| 3 | 442 | 305 | 300 | 148 |
| 4 | 509 | 350 | 335 | 215 |
| 5 | 547 | 384 | 370 | 286 |
| Rate of Drug Transfer (micrograms/$cm^2$/hour): | 15.20 | 10.72 | 10.00 | 8.67 |

The results shown in Table II indicate that the hydroxyethylmethacrylate polymer affords the most effective delivery of minoxidil with a rate of drug transfer of 15.20 micrograms per $cm^2$ per hour, as compared to 10.72 for sodium CMC, 10.00 for xanthan gum and 8.67 for Carbopol 940. Thus, the hydroxyethy1 methacrylate polymer exhibited a 42 to 75 percent higher drug delivery rate than the other systems tested.

In view of the foregoing Examples 1 and 2, hydroxyethylmethacrylate polymer is deemed to be highly effective in both confining the minoxidil formulation to the treatment area without excessive seepage and in delivering said minoxidil for better penetration at the treatment site. Similarly advantageous results are obtained with hydroxyethylacrylate polymer.

## Example 3

The following aqueous alcoholic formulations of minoxidil are prepared by admixture of the indicated percentages of ingredients.

### Table III

| Formulation: | A | B | C | D | E |
|---|---|---|---|---|---|
| Minoxidil (% w/v) . . . . . . | 0.5 | 5.0 | 1.0 | 3.0 | 2.0 |
| Hydroxyethyl methacrylate polymer (% w/v) . . | 1.0 | 5.0 | 10.0 | | |
| Hydroxyethyl acrylate polymer (% w/v) | | | | 3.0 | 5.0 |
| Propylene glycol (% w/v) | 10.0 | | | 5.0 | |
| 20:80 HOH:EtOH q.s. . . | 100.0 | | | | |
| 80:20 HOH:EtOH q.s. . . | | 100.0 | | | |
| 40:60 HOH:EtOH q.s. . . | | | 100.0 | | |
| 60:40 HOH:EtOH q.s. . . | | | | 100.0 | |
| 50:50 HOH:iPrOH q.s. . . | | | | | 100.0 |

Each of the foregoing formulations may be applied at the site of hair loss to patients afflicted with alopecia areata to provide effective delivery of minoxidil at the treatment side without substantial seepage.

CLAIMS

1. Hydroalcoholic composition containing an effective hair regrowing amount of minoxidil in an aqueous ethanolic or isopropanolic solvent, characterized by the inclusion of from about 1 to about 10 percent of hydroxy (lower alkyl) acrylate polymer, hydroxy (lower alkyl) methacrylate polymer or mixtures thereof.

2. The composition of Claim 1 which contains from about 0.5 to about 5.0 percent of minoxidil in an aqueous ethanolic solvent comprising from about 20 to about 80 percent of ethanol.

3. Hydroalcoholic composition of claim 1, characterized by the inclusion of from about 1 to about 10 percent of hydroxyethylmethacrylate homopolymer.

4. Hydroalcoholic composition containing from about 0.5 to about 5.0 percent of minoxidil in an aqueous ethanolic solvent comprising from about 20 to about 80 percent of ethanol, characterized by the inclusion of from about 1 to about 10 percent of hydroxyethylmethacrylate homopolymer having the formula:

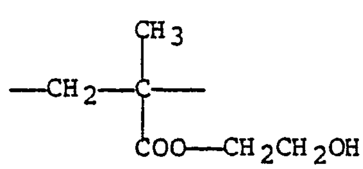

wherein n is the number of recurring monomeric units ranging from about 70 to about 400.

5. The composition of Claim 4 wherein the amount of the minoxidil is from about 1 to about 3 percent and the ethanol is from about 40 to about 60 percent.

6. The composition of Claim 4 wherein the amount of the minoxidil is from about 1 to about 3 percent and the ethanol is about 50 percent.

7. The composition of Claim 4 wherein the amount of hydroxyethylmethacrylate homopolymer is from about 3 to about 5 percent.

8. Hydroalcoholic composition containing from about 0.5 to about 5.0 percent of minoxidil in an aqueous ethanolic solvent comprising from about 40 to about 60 percent of ethanol, characterized by the inclusion of from about 3 to about 5 percent of hydroxyethylmethacrylate homopolymer having the formula:

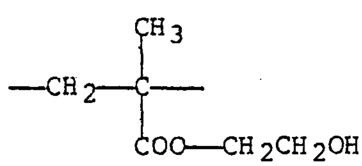

wherein n is from about 70 to about 400.

9. Use of the compositions of any of claims 1 to 8 for the preparation of topical hair regrowth compositions.

European Patent Office

# EUROPEAN SEARCH REPORT

0188793
Application number
EP 85 11 6389

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
| --- | --- | --- | --- |
| | | | A 61 K 7/06<br>A 61 K 31/505 |
| Y | US-A-4 139 619 (CHIDSEY)<br>* Claims; column 1, line 35 - column 2, line 13; examples 1-4 *<br>--- | 1-9 | |
| Y | US-A-3 963 685 (ABRAHAMS)<br>* Claims; column 1, line 37 - column 2, line 22; column 3, lines 18-34 *<br>----- | 1-9 | |
| | | | TECHNICAL FIELDS SEARCHED (Int. Cl.4) |
| | | | A 61 K<br>C 07 D |

The present search report has been drawn up for all claims

| Place of search | Date of completion of the search | Examiner |
| --- | --- | --- |
| THE HAGUE | 24-03-1986 | WILLEKENS G.E.J. |

CATEGORY OF CITED DOCUMENTS

- X : particularly relevant if taken alone
- Y : particularly relevant if combined with another document of the same category
- A : technological background
- O : non-written disclosure
- P : intermediate document
- T : theory or principle underlying the invention
- E : earlier patent document, but published on, or after the filing date
- D : document cited in the application
- L : document cited for other reasons
- & : member of the same patent family, corresponding document